# EUROPEAN PATENT APPLICATION

(11) **EP 4 691 399 A1**
(43) Date of publication of application: **11.02.2026**
(21) Application number: 24382898.5
(22) Date of filing: 09.08.2024
(51) Int. Cl.: A61B 18/14, A61B 17/295, A61B 90/00, A61B 17/29

(54) **END EFFECTOR WITH TISSUE POSITION SENSOR AND RELATED METHODS**

(71) Applicant: Cilag GmbH International, 6300 Zug (CH)
(72) Inventor: De Córdoba Matilla, José Luis, Cincinnati, 45242 (US)
(74) Representative: Carpmaels & Ransford LLP

(57) **Abstract**

Systems and methods are described for a surgical instrument. Certain embodiments can comprise an end effector with upper and lower jaws that can compress tissue therebetween. A sensing probe with a sensing electrode can be extended along the surface of the tissue in a track located within one of the jaws. The sensing electrode can be configured to detect location or type of tissue. Once position, size, or other factors are known about the tissue, a knife member can be deployed in the track or a separate track with increased accuracy and precision.

## Description

### BACKGROUND

A variety of ultrasonic surgical instruments include an end effector having a blade element to cut and/or seal tissue (e.g., by denaturing proteins in tissue cells). Some instruments are operable to seal tissue by applying radiofrequency (RF) electrosurgical energy to the tissue. Examples of such devices and related concepts are disclosed in U.S. Pat. No. 7,354,440, entitled "Electrosurgical Instrument and Method of Use," issued April 8, 2008, the disclosure of which is incorporated by reference herein; U.S. Pat. No. 7,381,209, entitled "Electrosurgical Instrument," issued June 3, 2008, the disclosure of which is incorporated by reference herein.

Some instruments are capable of applying both ultrasonic energy and RF electrosurgical energy to tissue. Examples of such instruments are described in U.S. Patent No. 9,949,785, entitled "Ultrasonic Surgical Instrument with Electrosurgical Feature," issued April 24, 2018, the disclosure of which is incorporated by reference herein; and U.S. Patent No. 8,663,220, entitled "Ultrasonic Electrosurgical Instruments," issued March 4, 2014, the disclosure of which is incorporated by reference herein.

While several surgical instruments and systems have been made and used, it is believed that no one prior to the inventors has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an exemplary electrosurgical instrument;
FIG. 2 depicts a perspective view of an exemplary articulation assembly and a first exemplary end effector of the electrosurgical instrument of FIG. 1;
FIG. 3 depicts an exploded view of the articulation assembly and end effector of FIG. 2;
FIG. 4 depicts a perspective view of the end effector that of FIG. 2;
FIG. 5 depicts an exploded perspective view of the end effector of FIG. 2;
FIG. 6 depicts an enlarged, side sectional view of a second exemplary end effector with an example of a sensing probe;
FIG. 7 depicts a partially exploded perspective view of a third exemplary end effector having various features removed for clarity;
FIG. 8 depicts an enlarged side view of another example of a sensing probe;
FIG. 9 depicts a sectional view of a fourth exemplary end effector;
FIG. 10A depicts a sectional view of a fifth exemplary end effector and shaft assembly;
FIG. 10B depicts an enlarged perspective sectional view of the end effector of FIG. 10A;
FIG. 11 illustrates a flow chart of an exemplary method of determining a position of a tissue; and
FIG. 12 illustrates a flow chart of a second exemplary method of manufacturing a surgical instrument.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a human or robotic operator of the surgical instrument. The term "proximal" refers to the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. In addition, the terms "upper," "lower," "top," and "bottom," are used with respect to the examples and associated figures and are not intended to unnecessarily limit the invention described herein.

### I. Example Electrosurgical Instrument

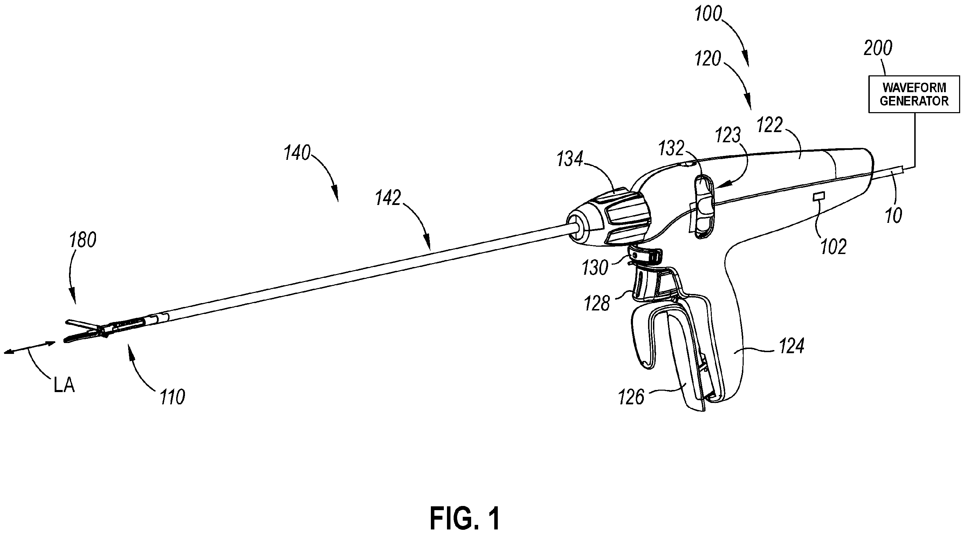

FIGS. 1-5 show a surgical system (98) including an exemplary electrosurgical instrument (100). As best seen in FIG. 1, electrosurgical instrument (100) includes a handle assembly (120), a shaft assembly (140), an articulation assembly (110), which may also be referred to as an articulation section (110), and a first exemplary end effector (180). As will be described in greater detail below, end effector (180) of electrosurgical instrument (100) is operable to grasp, cut, and seal or weld tissue (e.g., a blood vessel, etc.). In this example, end effector (180) is configured to apply a non-therapeutic bipolar radio frequency (RF) energy in order to identify and/or verify that the correct tissue is present in the end effector such that a therapeutic RF energy can be applied to seal or weld tissue. However, it should be understood that electrosurgical instrument (100) may be configured to seal or weld tissue through any other suitable means that would be apparent to one skilled in the art in view of the teachings herein. For example, electrosurgical instrument (100) may be configured to seal or weld tissue via an ultrasonic blade, staples, etc. In the present example, electrosurgical instrument (100) is electrically coupled to a waveform generator (200) of surgical system (98), which is capable of delivering therapeutic and non-therapeutic energy, via power cable (10).

Waveform generator (200) may be configured to provide all or some of the electrical power requirements for use of electrosurgical instrument (100). Any suitable waveform generator (200) may be used as would be apparent to one skilled in the art in view of the teachings herein. By way of non-limiting example, the waveform generator (200) may be constructed in accordance with at least some of the teachings of U.S. Pat. No. 8,986,302, entitled "Surgical Generator for Ultrasonic and Electrosurgical Devices," issued March 24, 2015, the disclosure of which is incorporated by reference herein, in its entirety. While in the current example, electrosurgical instrument (100) is coupled to waveform generator (200) via power cable (10), electrosurgical instrument (100) may contain an internal power source or plurality of power sources, such as a battery and/or supercapacitors, to electrically power electrosurgical instrument (100). Of course, any suitable combination of power sources may be utilized to power electrosurgical instrument (100) as would be apparent to one skilled in the art in view of the teaching herein. The power source may be configured to provide all or some of the electrical power requirements for use of electrosurgical instrument (110). By way of example only, the power source may be constructed in accordance with at least some of the teachings of U.S. Patent No. 8,986,302, entitled "Surgical Generator for Ultrasonic and Electrosurgical Devices," issued March 24, 2015, the disclosure of which is incorporated by reference herein.

Handle assembly (120) is configured to be grasped by an operator with one hand, such that an operator may control and manipulate electrosurgical instrument (100) with a single hand. Although electrosurgical instrument (100) is primarily described herein as being used by a human user, it should be noted that alternative versions exist in which one or more robotic systems (e.g., a robotic arm) may be used to control and manipulate electrosurgical instrument (100). Shaft assembly (140) extends distally from handle assembly (120) and connects to articulation assembly (110). Articulation assembly (110) is also connected to a proximal end of end effector (180). As will be described in greater detail below, components of handle assembly (120) are configured to control end effector (180) such that an operator may grasp, cut, and seal or weld tissue. Articulation assembly (110) is configured to deflect end effector (180) from the longitudinal axis (LA) defined by shaft assembly (140).

Handle assembly (120) of the present example includes a control unit (102) housed within a body (122), a pistol grip (124), a jaw closure trigger (126), a knife trigger (128), an activation button (130), an articulation control (132), and a knob (134). As will be described in greater detail below, jaw closure trigger (126) may be pivoted toward and away from pistol grip (124) and/or body (122) to open and close jaws (182, 184) of end effector (180) to grasp tissue. Additionally, knife trigger (128) may be pivoted toward and away from pistol grip (124) and/or body (122) to actuate a knife member (176) within the confines of jaws (182, 184) to cut tissue captured between jaws (182, 184). Further, activation button (130) may be pressed to apply radio frequency (RF) energy to tissue via electrodes (194, 196) of jaws (182, 184), respectively. In some versions, electrodes (194, 196) of jaws (182, 184) are in a bifurcation configuration where electrodes (194, 196) move relative to a central axis and nearly equal and opposite to one another. Control unit (102), body (122), pistol grip (124), jaw closure trigger (126), knife trigger (128), activation button (130), articulation control (132), and knob (134) may provide functionality in accordance with at least some of the teachings of U.S. Pat. No. 9,949,785, entitled "Ultrasonic Surgical Instrument with Electrosurgical Feature," issued April 24, 2018, the disclosure of which is incorporated by reference herein.

Body (122) of handle assembly (120) defines an opening (123) through which a portion of articulation control (132) protrudes. Articulation control (132) is rotatably disposed within body (122) such that an operator may rotate the portion of articulation control (132) protruding from opening (123) to rotate the portion of articulation control (132) located within body (122). Rotation of articulation control (132) relative to body (122) will bend articulation assembly (110) in order to drive deflection of end effector (180) from the longitudinal axis (LA) defined by shaft assembly (140). Articulation control (132) and articulation assembly (110) may include any suitable features to drive deflection of end effector (180) from the longitudinal axis (LA) defined by shaft assembly (140) as would be apparent to one skilled in the art in view of the teachings herein.

Knob (134) is rotatably disposed on the distal end of body (122) and is configured to rotate end effector (180), articulation assembly (110), and shaft assembly (140) about the longitudinal axis (LA) of shaft assembly (140) relative to handle assembly (120). While in the current example, end effector (180), articulation assembly (110), and shaft assembly (140) are rotated by knob (134), knob (134) may be configured to rotate end effector (180) and articulation assembly (110) relative to selected portions of shaft assembly (140). Knob (134) may include any suitable features to rotate end effector (180), articulation assembly (110), and shaft assembly (140) as would be apparent to one skilled in the art in view of the teachings herein.

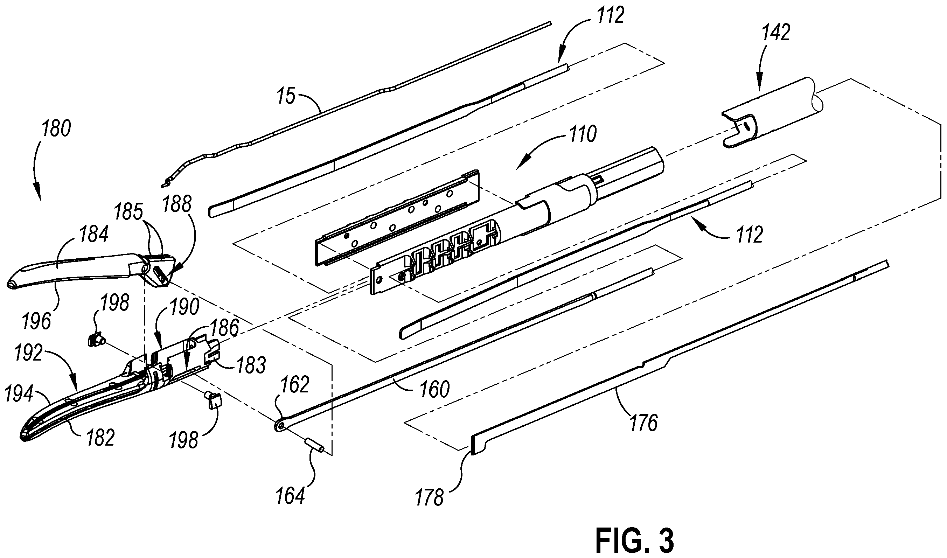

Shaft assembly (140) includes distal portion (142) extending distally from handle assembly (120) and a proximal portion housed within the confines of body (122) of handle assembly (120). Referring to FIG. 3, shaft assembly (140) houses a jaw closure connector (160) that couples jaw closure trigger (126) with end effector (180). Additionally, shaft assembly (140) houses a portion of knife member (176) extending between distal a distal cutting edge (178) of knife member (176) and knife trigger (128). Shaft assembly (140) also houses actuating members (112) that couple articulation assembly (110) with articulation control (132); as well as an electrical coupling (15) that operatively couples electrodes (194, 196) with activation button (130). As will be described in greater detail below, jaw closure connector (160) is configured to translate relative to shaft assembly (140) to open and close jaws (182, 184) of end effector (180); while knife member (176) is coupled to knife trigger (128) of handle assembly (120) to translate distal cutting edge (178) within the confines of end effector (180); and activation button (130) is configured to activate electrodes (194, 196).

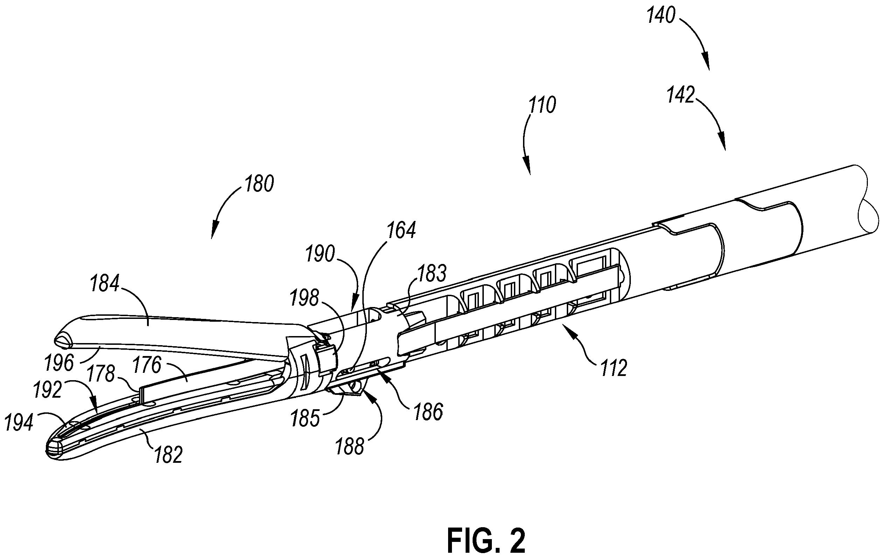

As best seen in FIGS. 2-5, end effector (180) includes lower jaw (182) pivotally coupled with upper jaw (184) via pivot couplings (198). Lower jaw (182) includes a proximal body (183) defining a slot (186), while upper jaw (184) includes proximal arms (185) defining a slot (188). Lower jaw (182) also defines a central channel (190) that is configured to receive proximal arms (185) of upper jaw (184), portions of knife member (176), jaw closure connector (160), and pin (164). Slots (186, 188) each slidably receive pin (164), which is attached to a distal coupling portion (162) of jaw closure connector (160). Additionally, lower jaw (182) includes a force sensor (195) located at a distal tip of lower jaw (182), though force sensor (195) may alternatively be positioned at any other suitable location. Force sensor (195) may be in communication with control unit (102). Force sensor (195) may be configured to measure the closure force generated by pivoting jaws (182, 184) into a closed configuration in accordance with the description herein. Additionally, force sensor (195) may communicate this data to control unit (102). Any suitable components may be used for force sensor (195) as would be apparent to one skilled in art in view of the teachings herein. For example, force sensor (195) may take the form of a strain gauge. In some variations, end effector (180) includes more than one force sensor.

While in the current example, a force sensor (195) is incorporated into electrosurgical instrument (100) and is in communication with control unit (102), any other suitable sensors or feedback mechanisms may be additionally or alternatively incorporated into electrosurgical instrument (100) while in communication with control unit (102) as would be apparent to one skilled in the art in view of the teachings herein. For instance, an articulation sensor or feedback mechanism may be incorporated into electrosurgical instrument (100), where the articulation sensor communicates signals to control unit (102) indicative of the degree end effector 180 is deflected from the longitudinal axis (LA) by articulation control (132) and articulation assembly (110).

As will be described in greater detail below, jaw closure connector (160) is operable to translate within central channel (190) of lower jaw (182). Translation of jaw closure connector (160) drives pin (164). As will also be described in greater detail below, with pin (164) being located within both slots (186, 188), and with slots (186, 188) being angled relative to each other, pin (164) cams against proximal arms (185) to pivot upper jaw (184) toward and away from lower jaw (182) about pivot couplings (198). Therefore, upper jaw (184) is configured to pivot toward and away from lower jaw (182) about pivot couplings (198) to grasp tissue.

The term "pivot" does not necessarily require rotation about a fixed axis and may include rotation about an axis that moves relative to end effector (180). Therefore, the axis at which upper jaw (184) pivots about lower jaw (182) may translate relative to both upper jaw (184) and lower jaw (182). Any suitable translation of the pivot axis may be used as would be apparent to one skilled in the art in view of the teachings herein.

Lower jaw (182) and upper jaw (184) also define a knife pathway (192). Knife pathway (192) is configured to slidably receive knife member (176), such that knife member (176) may be retracted, and advanced, to cut tissue captured between jaws (182, 184).

Lower jaw (182) and upper jaw (184) each comprise respective electrodes (194, 196). The power source may provide RF energy to electrodes (194, 196) via electrical coupling (15) that extends through handle assembly (120), shaft assembly (140), articulation assembly (110), and electrically couples with one or both of electrodes (194, 196). Electrical coupling (15) may selectively activate electrodes (194, 196) in response to an operator pressing activation button (130). In some instances, control unit (102) may couple electrical coupling (15) with activation button (130), such that control unit (102) activates electrodes (194, 196) in response to operator pressing activation button (130). Control unit (102) may have any suitable components in order to perform suitable functions as would be apparent to one skilled in the art in view of the teachings herein. For instance, control unit (102) may have a processor, memory unit, suitable circuitry, etc. Examples of features and functionalities that may be incorporated into control unit (102) will be described in greater detail below. The control of ultrasonic energy and/or RF electrosurgical energy may be provided in accordance with at least some of the teachings of U.S. Patent No. 8,663,220, entitled "Ultrasonic Electrosurgical Instruments," issued March 4, 2014, the disclosure of which is incorporated by reference herein; and/or U.S. Pat. No. 9,949,785, entitled "Ultrasonic Surgical Instrument with Electrosurgical Feature," issued April 24, 2018, the disclosure of which is incorporated by reference herein.

As described above, jaw closure trigger (126) may be pivoted toward and away from pistol grip (124) and/or body (122) to open and close jaws (182, 184) of end effector (180) to grasp tissue. In particular, as will be described in greater detail below, pivoting jaw closure trigger (126) toward pistol grip (124) may proximally actuate jaw closure connector (160) and pin (164), which in turn cams against slots (188) of proximal arms (185) of upper jaw (184), thereby rotating upper jaw (184) about pivot couplings (198) toward lower jaw (182) such that jaws (182, 184) achieve a closed configuration.

In some versions, knife trigger (128) may be pivoted toward and away from body (122) and/or pistol grip (124) to actuate knife member (176) within knife pathway (192) of jaws (182, 184) to cut tissue captured between jaws (182, 184). In particular, handle assembly (120) further includes a knife coupling body that is slidably coupled along proximal portion of shaft assembly (140). Knife coupling body is coupled with knife member (176) such that translation of knife coupling body relative to proximal portion of shaft assembly (140) translates knife member (176) relative to shaft assembly (140).

In another version, knife coupling body may be coupled to a knife actuation assembly such that as knife trigger (128) pivots toward body (122) and/or pistol grip (124), knife actuation assembly drives knife coupling body distally, thereby driving knife member (176) distally within knife pathway (192). Because knife coupling body is coupled to knife member (176), knife member (176) translates distally within shaft assembly (140), articulation assembly (110), and within knife pathway (192) of end effector (180). Knife member (176) includes distal cutting edge (178) that is configured to sever tissue captured between jaws (182, 184). Therefore, pivoting knife trigger (128) causes knife member (176) to actuate within knife pathway (192) of end effector (180) to sever tissue captured between jaws (182, 184).

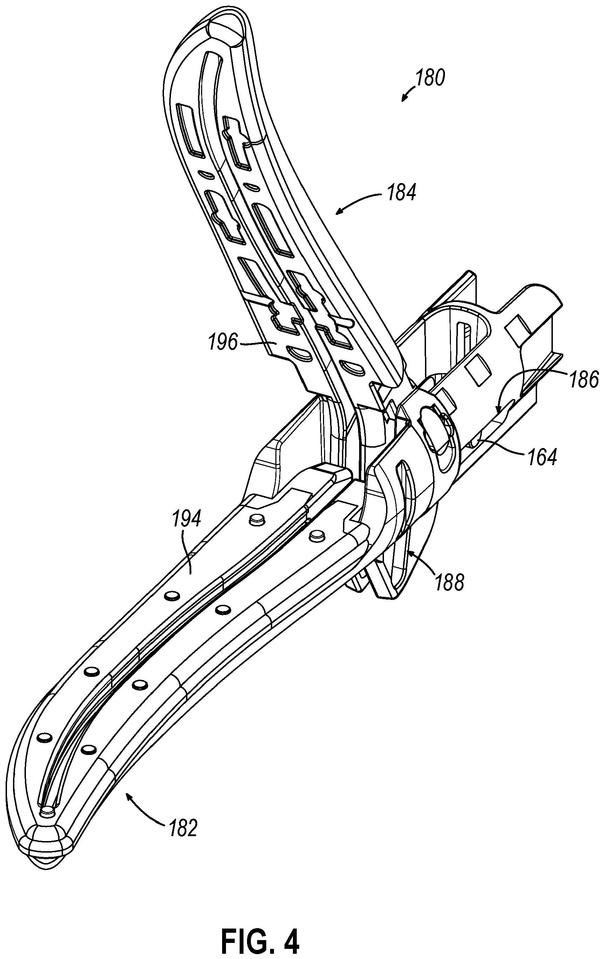

Electrosurgical instrument (110) may generate heat as end effectors (180) seal and/or cut tissue. The energized feature can include at least one of knife member (176) shown in FIGS. 2-3, electrodes (194, 196) shown in FIG. 4, or another suitable energized feature. Energized features, e.g., knife member (176) or electrodes (194, 196) can include a surface that is configured to contact the tissue. With distal cutting edge (178) of knife member (176) actuated to the advanced position, an operator may press activation button (130) to selectively activate electrodes (194, 196) of jaws (182, 184) to seal or weld severed tissue captured between jaws (182, 184). It should be understood that the operator may also press activation button (130) to selectively activate electrodes (194, 196) of jaws (182, 184) at any suitable time during exemplary use. Therefore, the operator may also press activation button (130) while knife member (176) is retracted. Next, the operator may release jaw closure trigger (126) such that jaws (182, 184) pivot into the opened configuration, releasing tissue.

### II. Sensing Tissue for Determinations of Tissue State

Electrosurgical instrument (100) discussed above is configured to clamp tissue using end effector (180). Once securely clamped, electrodes (194, 196) in end effector (180) can apply a non-therapeutic (i.e., low voltage) waveform to the tissue; and sensor devices measure the returning waveform to calculate and measure the impedance of the tissue. In one example, one or more electrodes (194, 196) are operatively connected to such sensor devices such that electrodes (194, 196) may be referred to as sensors in this respect. More specifically, electrosurgical instrument (100), via one or more sub-circuits, will provide non-therapeutic energy to the extracellular and intracellular fluid present within a given (e.g., clamped) region of tissue to determine a phase and a magnitude of the impedance of the tissue within jaws (182, 184). A processor may then relay information associated with a state of the tissue, such as, for example, tissue type, tissue phase, tissue margin, and the like. Using this associated information, a system inclusive of such electrosurgical instrument (100) cannot only verify that the proper tissue is clamped between jaws (182, 184), but can also determine if any non-tissue material is present between jaws (182, 184), and/or if a proper seal has been created after applying the therapeutic RF energy.

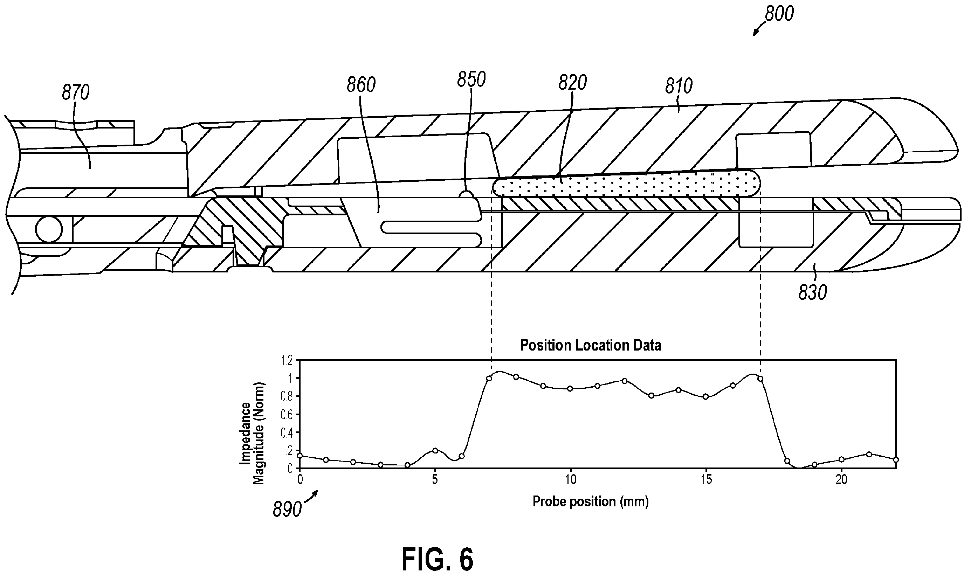

Additionally, in a second exemplary end effector (800) shown in FIG. 6, tissue position relative to jaws (810, 830) is detected for additional tissue sensing capabilities. Jaws (810, 830) can close, or approximately close, about tissue (820) with end effector (800) similar to end effector (180) discussed above unless as otherwise described herein. To this end, sensing probe (860) is retractable and comprises a sensing electrode (850). As jaws (810, 830) close on, or closely abut, tissue (820), sensing electrode (850) can be moved along the underside (in this view) of tissue (820). Sensing electrode (850), via sensing probe (860) may be coupled to other components of electrosurgical instrument (100), such as control unit (102) (*see* FIG. 1) in the present example. With sensing electrode (850), end effector (800) is configured to apply a waveform (e.g., non-therapeutic or low voltage) to tissue (820). Control unit (102), in turn, analyzes the returning waveform to calculate and measure, e.g.: the impedance of the tissue (850); detect where tissue (850) begins and ends relative to jaws (810, 830); detect types or conditions of tissue; or other characteristics. For example, impedance graph (890) displays impedance magnitude (normalized) against a position of sensing probe (860). As can be seen, impedance magnitude jumps when sensing electrode (850) is in contact with tissue (820). In one example, this may help a user and/or electrosurgical instrument (100) to know the precise location of tissue (820) clamped between jaws (810, 830) and allow for greater precision in any cutting procedure with knife member (870). In this view, knife member (870) is shown retracted, prior to a cutting procedure.

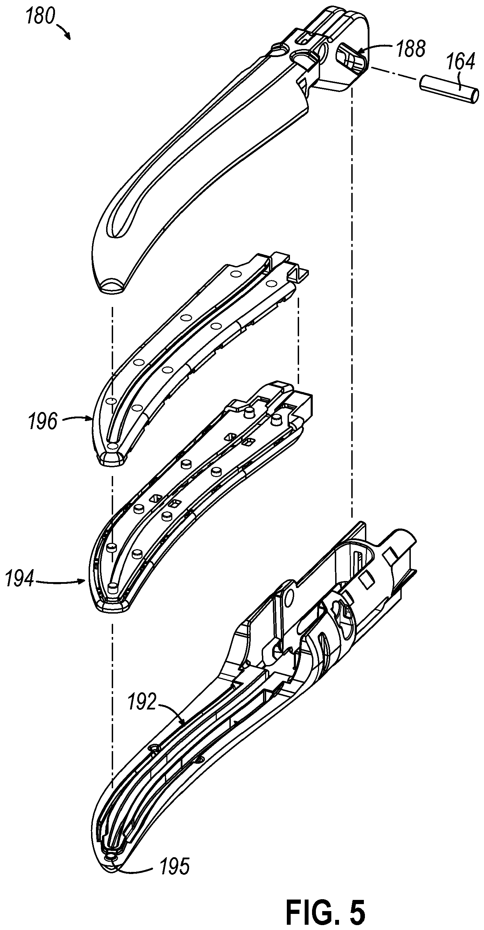

Notably, the sensing probe (860) is received and configured to translate within knife pathway (192) (*see* FIG. 5). In this respect, sensing probe (860) shares knife pathway (192) (*see* FIG. 5) with knife member (870) such that sensing probe (860) first translates along knife pathway (192) without knife member (870) received in knife pathway (192) followed by knife member (870) translating along knife pathway (192) without sensing probe (860) received in knife pathway (192). In this respect, sensing probe (860) and knife member (870) translate along knife pathway (192) in succession without simultaneously being positioned in knife pathway (192) between clamped jaws (810, 830). In another example, sensing probe (860) and knife member (870) may be able to translating simultaneously along a shared knife pathway (192) or, in yet another example, sensing probe (860) and knife member (870) may travel along respective pathways such that the invention is not intended to be unnecessarily limited to the particular arrangement of sensing probe (860) and knife member (870) as shown in the present example.

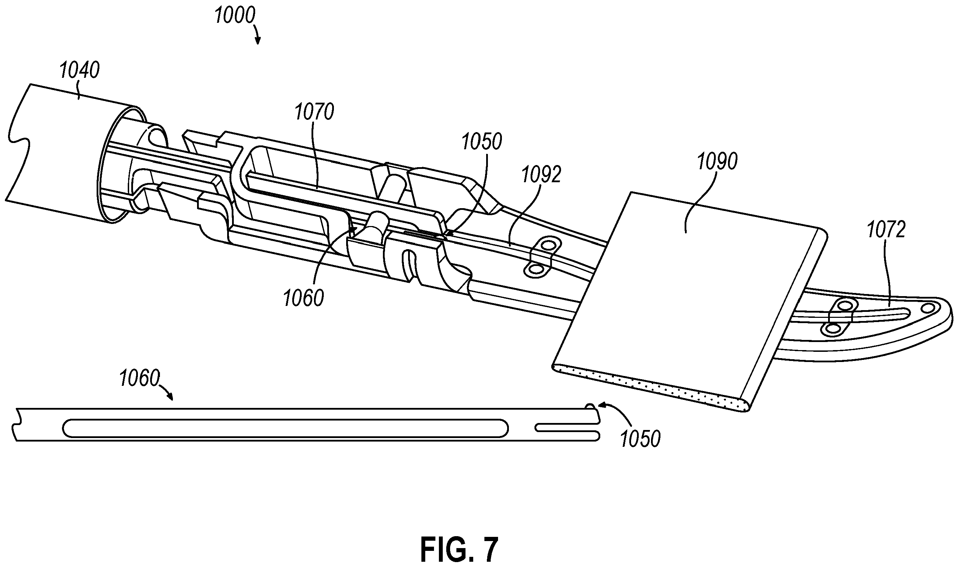

FIG. 7 illustrates a third exemplary end effector (1000) similar to end effectors (180, 800) discussed above unless otherwise described herein. Tissue (1080) is located on top of jaw (1070) (a top jaw is not shown in this view) and knife member (1070) and sensing probe (1060) are shown retracted into shaft assembly (1040). One or more pathways (1092) can be incorporated into end effector (1000) and jaw (1070) to form a path for the deployment of knife member (1070) and sensing probe (1060). As can be seen, it may be preferable for sensing probe (1060) to sit lower in the shaft assembly (1040) and/or pathway (1092). This can allow for sensing probe (1060) to be deployed along an underside of tissue (1080) while further inhibiting damage to tissue (1080). In this view, sensing probe (1060), with sensing electrode (1050) is shown to move along an underside of tissue (1080). In other embodiments, sensing probe (1060) and sensing electrode (1050) could be made to move along a topside of tissue (1080), such as by deploying along a pathway in another jaw of end effector (1000).

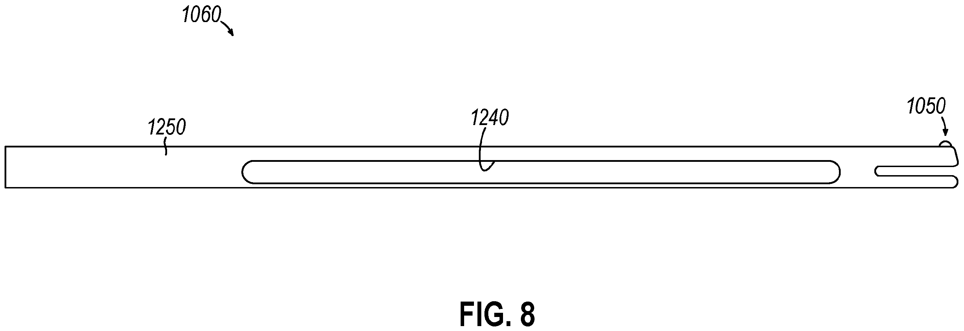

Another view of sensing probe (1060) embodiment is shown in FIG. 8. Sensing probe (1060) comprises sensing electrode (1050), a slot (1240), and probe body (1250). Sensing probe (1060) can comprise various types of metals to allow for electrical coupling between sensing electrode (1050) and e.g., control unit (102) (*see* FIG. 1). Slot (1240) and probe body (1250) are configured to be engaged and direct in order to manipulate sensing probe (1060) for use e.g., via actuators and/or stops/brakes within e.g., end effector (1000) of FIG. 7 or shaft assembly (140) of FIG. 2. For example, an actuator may deploy sensing probe (1060) by pressing on a distal end of probe body (1250). Ridges, notches, protrusions, brakes, or other stop elements within a shaft assembly may be located within slot (1240) and may prevent deployment of sensing probe (1060) beyond certain distances by engaging the inner surface of slot (1240), thus preventing further movement (left or right in the view of FIG. 8) of the sensing probe (1060). Other means of actuation and braking of sensing probe (1060) are possible. For example, a sensing probe (1060) could comprise multiple slots (1240) that engage different elements of a shaft assembly. Further description is given below of potential deployment mechanisms.

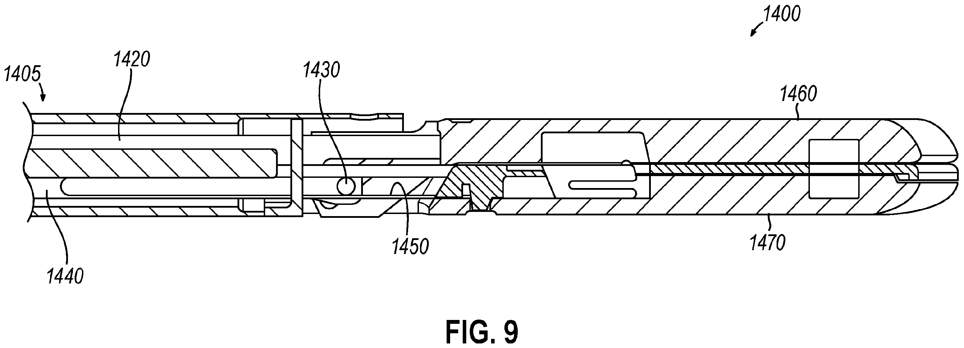

FIG. 9 shows a fourth exemplary end effector (1400) similar to end effectors (180, 800, 1000) discussed above unless otherwise described herein. Upper jaw (1460) and lower jaw (1470) are shown in a closed position, with portions of end effector (1400) and shaft assembly (1405). Knife member (1420) and sensing probe (1440) can be seen. Slot (1450) can be seen in sensing probe (1440). Stop element (1430) passes through slot (1450) and slot (1450) can be sized such that it can be deployed and retracted appropriate distances for the desired use case, with stop element (1430) stopping further displacement of sensing probe (1440) by engaging the ends of slot (1450). Stop element (1430) can be configured to also pass through a slot on knife member (1420) and provide similar stopping functionality or stop element (1430) may be for manipulation of sensing probe (1440). Stop element (1430) can comprise other components of end effector (1400) or shaft assembly (1405). For example, stop element (1430) can comprise pivot couplings (198) (*see* FIG. 2), which provides pivot capabilities with respect to upper jaw (1460) and lower jaw (1470). Having stop element (1430) comprise pivot couplings (198) (*see* FIG. 2) can result in fewer parts, and potentially better lifespan for end effectors and shaft assemblies as described herein.

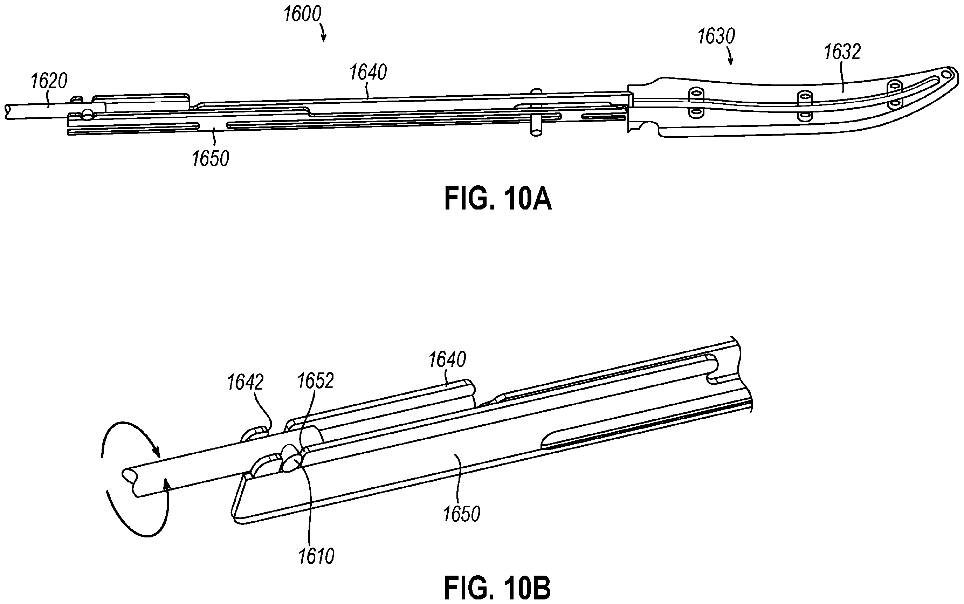

FIGS. 10A-10B illustrate a fifth exemplary end effector (1600) similar to end effectors (180, 800, 1000, 1400) discussed above unless otherwise described herein. FIGS. 10A-10B illustrate one possible deployment mechanism for selectively deploying knife member (1640) or sensing probe (1650). With respect to FIG. 10A, knife member (1640) and sensing probe (1650) are in a retracted position. Deployer rod (1620) sits between knife member (1640) and sensing probe (1650) and comprises an extension (1610). As deployer rod (1620) rotates the extension (1610) to move between a first indentation (1642) on knife member (1640) and a second indentation (1652) on sensing probe (1650). Once engaged in the first or second indentation (1642, 1652), the deployer rod (1620) can be pressed forward and backward to deploy or retract the respective knife member (1640) or sensing probe (1650). Deployer rod (1620) can be controlled by e.g., control unit (102) (*see* FIG. 1) and/or other buttons or triggers on electrosurgical instrument (100). In one example, a user may manipulate the control unit (102) or deployer rod (1620) to engage the second indentation (1652) and deploy the sensing probe (1650). The user may use the sensing probe (1650) to move the sensing electrode under/above/along tissue so as to detect e.g., tissue size, type or other features. The user may then retract the sensing probe (1650), and then manipulate the deployer rod (1620) to rotate and engage the first indentation (1642) of knife member (1640). The user may then deploy knife member (1640) with deployer rod (1620) to perform a cut or incision. The length of deployment may be based at least in part on measurements taken using sensing probe (1650) and its sensing electrode.

### III. Method of Use

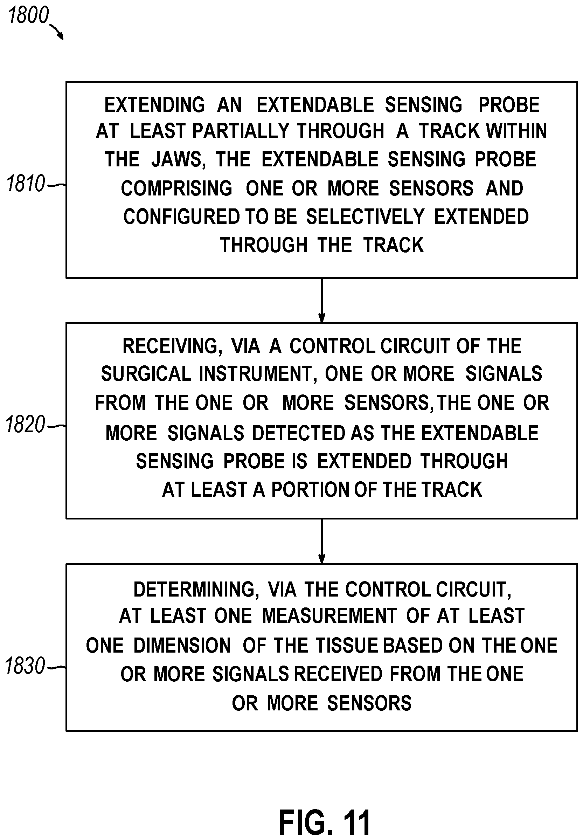

One possible method (1800) of manipulating tissue positioned between jaws of an end effector of a surgical instrument (100) is shown in FIG. 11. At step (1810), method (1800) includes extending an extendable sensing probe at least partially through a track within the jaws, the extendable sensing probe comprising one or more sensors and configured to be selectively extended through the track. At step (1820), method (1800) includes receiving, via a control circuit of the surgical instrument, one or more signals from the one or more sensors, the one or more signals detected as the extendable sensing probe is extended through at least a portion of the track. At step (1830), method (1800) includes determining, via the control circuit, at least one measurement of at least one dimension of the tissue based on the one or more signals received from the one or more sensors. Method (1800) can comprise a variety of additional, alternative, or optional steps or other variations.

### IV. Method of Manufacturing

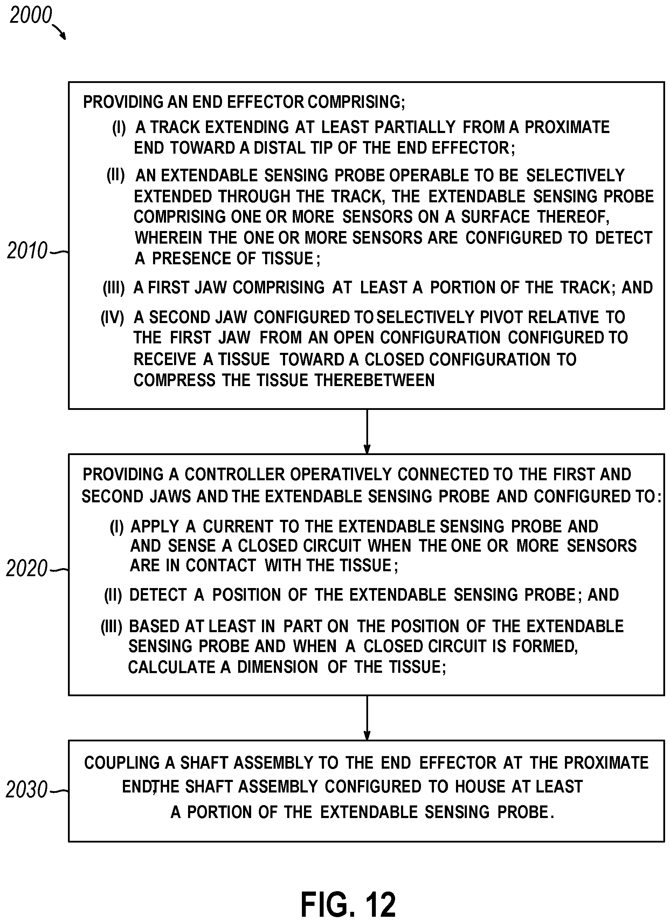

FIG. 12 shows a diagrammatic view of one possible method (2000) of manufacturing a surgical instrument. At step (2010), method (2000) includes providing an end effector comprising; (i) a track extending at least partially from a proximate end toward a distal tip of the end effector; (ii) an extendable sensing probe operable to be selectively extended through the track, the extendable sensing probe comprising one or more sensors on a surface thereof, wherein the one or more sensors are configured to detect a presence of tissue; (iii) a first jaw comprising at least a portion of the track; and (iv) a second jaw configured to selectively pivot relative to the fist jaw from an open configuration configured to receive a tissue toward a closed configuration to compress the tissue therebetween. At step (2020), method (2000) includes providing a controller operatively connected to the first and second jaws and the extendable sensing probe and configured to: (i) apply a current to the extendable sensing probe and sense a closed circuit when the one or more sensors are in contact with the tissue; (ii) detect a position of the extendable sensing probe; and (iii) based at least in part on the position of the extendable sensing probe and when a closed circuit is formed, calculate a dimension of the tissue. At step (2030), method (2000) includes coupling a shaft assembly to the end effector at the proximate end, the shaft assembly configured to house at least a portion of the extendable sensing probe. Method (2000) can comprise a variety of additional, alternative, or optional steps or other variations.

### V. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.
1. A surgical instrument, comprising:
   (a) a shaft assembly;
   (b) an end effector distally extending from the shaft assembly, including:
      (i) a first jaw,
      (ii) a second jaw configured to selectively move relative to the first jaw from an open configuration configured to receive a tissue toward a closed configuration to compress the tissue therebetween,
      (iii) a first track distally extending along the first jaw, and
      (iv) an extendable sensing probe comprising a sensor configured to detect a presence of the tissue, wherein the extendable sensing probe is configured to selectively move through the first track for detecting a presence of the tissue along the first jaw; and
   (c) a controller electrically connected to the extendable sensing probe and configured to determine a position of the tissue received between the first and second jaws in the closed configuration via the extendable sensing probe.
2. The surgical instrument of example 1, wherein the end effector further comprises a knife member configured to be selectively deployed for cutting the tissue.
3. The surgical instrument of example 2, wherein the knife member is configured to be selectively deployed through the first track.
4. The surgical instrument of example 2, wherein the end effector further comprises a second track and the knife member is configured to selectively move through the second track.
5. The surgical instrument of example 2, wherein the shaft assembly is configured to couple the end effector to a handle and configured to house at least a portion of the knife member and at least a portion of the extendable sensing probe, wherein the knife member comprises a first indentation and the extendable sensing probe comprises a second indentation, the shaft assembly comprising:
   a deployer rod positioned between the knife member and the extendable sensing probe and comprising an extension configured to engage the first and second indentations, wherein the deployer is configured to be rotatably manipulated to selectively engage the first or second indentations, wherein once engaged, the deployer rod is operable to selectively extend the knife member or extendable sensing probe.
6. The surgical instrument of example 2, wherein the shaft assembly is configured to couple the end effector to a handle and configured to house at least a portion of the knife member and at least a portion of the extendable sensing probe, wherein the knife member comprises a first longitudinal opening and the extendable sensing probe comprises a second longitudinal opening, the shaft assembly comprising one or more extensions configured to pass through the first and second longitudinal openings and configured to restrain at least some movement of the knife member and the extendable sensing probe within the shaft assembly.
7. The surgical instrument of example 1, wherein the controller is configured to:
   apply a current to the extendable sensing probe and sense a closed circuit when the one or more sensors are in contact with the tissue; and
   detect a position of the extendable sensing probe.
8. The surgical instrument of example 7, wherein the controller is further configured to, based at least in part on the position of the extendable sensing probe, and when a closed circuit is formed, calculate a dimension of the tissue.
9. The surgical instrument of example 1, wherein the extendable sensing probe is coated to be electrically isolated except at the sensor.
10. The surgical instrument of example 1, wherein the extendable sensing probe is configured to be an electro-cutter, and wherein the controller is further configured to selectively operate the extendable sensing probe to cut the tissue.
11. The surgical instrument of example 1, wherein the second jaw comprises at least a portion of the first track.
12. The surgical instrument of example 1, wherein the shaft assembly is configured to couple the end effector to a handle and configured to house at least a portion of the extendable sensing probe, wherein the extendable sensing probe comprises a longitudinal opening, the shaft assembly comprising one or more extensions configured to pass through the longitudinal opening and configured to restrain at least some movement of the extendable sensing probe within the shaft assembly.
13. A method of manipulating tissue positioned between jaws of an end effector of a surgical instrument, the method comprising:
   extending an extendable sensing probe at least partially through a track within the jaws, the extendable sensing probe comprising one or more sensors and configured to be selectively extended through the track;
   receiving, via a control circuit of the surgical instrument, one or more signals from the one or more sensors, the one or more signals detected as the extendable sensing probe is extended through at least a portion of the track; and
   determining, via the control circuit, at least one measurement of at least one dimension of the tissue based on the one or more signals received from the one or more sensors.
14. The method of example 13, further comprising characterizing, via the control circuit, the tissue based at least in part on the one or more signals.
15. The method of example 13, further comprising:
   retracting the extendable sensing probe; and
   extending a knife member to cut at least a portion of the tissue, wherein the extending is based at least in part on the at least one measurement.
16. The method of example 15, wherein the knife member is extended through the track.
17. The method of example 15, wherein the knife member is extended through a second track in the jaws.
18. The method of example 13, wherein the extendable sensing probe is configured to comprise an electro-cutter, and the method further comprises manipulating the extendable sensing probe to cut at least a portion of the tissue based at least in part on the at least one measurement.
19. A surgical instrument, comprising:
   (a) a shaft assembly;
   (b) an end effector distally extending from the shaft assembly, including:
      (i) one or more jaws configured to selectively move relative to each other from an open configuration configured to receive a tissue toward a closed configuration to compress the tissue therebetween,
      (ii) a track distally extending along one of the one or more jaws, and
      (iii) an extendable sensing probe configured to detect a presence of the tissue, wherein the extendable sensing probe is configured to selectively move through the track while the tissue is compressed between the one or more jaws; and
   (c) a controller electrically connected to the extendable sensing probe and configured to determine one or more characteristics of the tissue received between the one or more jaws in the closed configuration via the extendable sensing probe.
20. The surgical instrument of example 19, wherein the one or more characteristics comprise at least one of: a length; a width; a type; an impedance.

### VI. Miscellaneous

It should be understood that any of the versions of instruments described herein may include various other features in addition to or in lieu of those described above. By way of example only, any of the instruments described herein may also include one or more of the various features disclosed in any of the various references that are incorporated by reference herein. It should also be understood that the teachings herein may be readily applied to any of the instruments described in any of the other references cited herein, such that the teachings herein may be readily combined with the teachings of any of the references cited herein in numerous ways. Other types of instruments into which the teachings herein may be incorporated will be apparent to those of ordinary skill in the art.

It should also be understood that any ranges of values referred to herein should be read to include the upper and lower boundaries of such ranges. For instance, a range expressed as ranging "between approximately 1.0 inches and approximately 1.5 inches" should be read to include approximately 1.0 inches and approximately 1.5 inches, in addition to including the values between those upper and lower boundaries.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices described above may have application in conventional medical treatments and procedures conducted by a medical professional, as well as application in robotic-assisted medical treatments and procedures.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following examples and is understood not to be limited to the details of structure and operation shown and described in the specification and drawings.

## Claims

1. A surgical instrument, comprising:
(a) a shaft assembly;
(b) an end effector distally extending from the shaft assembly, including:
(i) one or more jaws configured to selectively move relative to each other from an open configuration configured to receive a tissue toward a closed configuration to compress the tissue therebetween,
(ii) a track distally extending along one of the one or more jaws, and
(iii) an extendable sensing probe configured to detect a presence of the tissue, wherein the extendable sensing probe is configured to selectively move through the track while the tissue is compressed between the one or more jaws; and
(c) a controller electrically connected to the extendable sensing probe and configured to determine one or more characteristics of the tissue received between the one or more jaws in the closed configuration via the extendable sensing probe.

2. The surgical instrument of Claim 1, wherein the one or more jaws comprises:
(i) a first jaw, and
(ii) a second jaw configured to selectively move relative to the first jaw;
wherein the track comprises a first track distally extending along the first jaw;
wherein the extendable sensing probe comprises a sensor and is configured to selectively move through the first track for detecting a presence of the tissue along the first jaw; and
wherein the controller is configured to determine a position of the tissue received between the first and second jaws.

3. The surgical instrument of Claim 1 or Claim 2, wherein the end effector further comprises a knife member configured to be selectively deployed for cutting the tissue.

4. The surgical instrument of Claim 3, wherein the knife member is configured to be selectively deployed through the first track.

5. The surgical instrument of Claim 3 or Claim 4, wherein the end effector further comprises a second track and the knife member is configured to selectively move through the second track.

6. The surgical instrument of any one or more of Claims 3 through 5, wherein the shaft assembly is configured to couple the end effector to a handle and configured to house at least a portion of the extendable knife member and at least a portion of the extendable sensing probe, wherein the extendable knife member comprises a first indentation and the extendable sensing probe comprises a second indentation, the shaft assembly comprising: a deployer rod positioned between the extendable knife member and the extendable sensing probe and comprising an extension configured to engage the first and second indentations, wherein the deployer is configured to be rotatably manipulated to selectively engage the first or second indentations, wherein once engaged, the deployer rod is operable to selectively extend the extendable knife member or extendable sensing probe.

7. The surgical instrument of any one or more of Claims 3 through 5, wherein the shaft assembly is configured to couple the end effector to a handle and configured to house at least a portion of the extendable knife member and at least a portion of the extendable sensing probe, wherein the extendable knife member comprises a first longitudinal opening and the extendable sensing probe comprises a second longitudinal opening, the shaft assembly comprising one or more extensions configured to pass through the first and second longitudinal openings and configured to restrain at least some movement of the extendable knife member and the extendable sensing probe within the shaft assembly.

8. The surgical instrument of any one or more of Claims 3 through 7, wherein the controller is configured to: apply a current to the extendable sensing probe and sense a closed circuit when the one or more sensors are in contact with the tissue; and detect a position of the extendable sensing probe.

9. The surgical instrument of Claim 8, wherein the controller is further configured to, based at least in part on the position of the extendable sensing probe, and when a closed circuit is formed, calculate a dimension of the tissue.

10. The surgical instrument of any one or more of Claims 3 through 9, wherein the extendable sensing probe is coated to be electrically isolated except at the sensor.

11. The surgical instrument of any one or more of Claims 3 through 10, wherein the extendable sensing probe is configured to be an electro-cutter, and wherein the controller is further configured to selectively operate the extendable sensing probe to cut the tissue.

12. The surgical instrument of any one or more of Claims 3 through 11, wherein the second jaw comprises at least a portion of the first track.

13. The surgical instrument of any one or more of Claims 3 through 12, wherein the shaft assembly is configured to couple the end effector to a handle and configured to house at least a portion of the extendable sensing probe, wherein the extendable sensing probe comprises a longitudinal opening, the shaft assembly comprising one or more extensions configured to pass through the longitudinal opening and configured to restrain at least some movement of the extendable sensing probe within the shaft assembly.

14. The surgical instrument of any preceding Claim, wherein the one or more characteristics comprise at least one of: a length; a width; a type; an impedance.
